# EUROPÄISCHE PATENTSCHRIFT

(11) **EP 4 045 097 B1**
(45) Veröffentlichungstag und Bekanntmachung des Hinweises auf die Patenterteilung: **15.05.2024**
(21) Anmeldenummer: 20781596.0
(22) Anmeldetag: 07.10.2020
(51) Int. Cl.: A61L 27/48, A61L 27/50, A61L 27/52, A61L 31/12, A61L 31/14

(54) **DICHTUNGSMATERIAL FÜR EIN MEDIZINISCHES IMPLANTAT**
SEALING MATERIAL FOR A MEDICAL IMPLANT
MATÉRIAU D'ÉTANCHÉITÉ POUR UN IMPLANT MÉDICAL

(30) Priorität: 17.10.2019 EP 19203801
(43) Veröffentlichungstag der Anmeldung: 24.08.2022
(73) Patentinhaber: Cortronik GmbH, 18119 Rostock-Warnemünde (DE)
(72) Erfinder: DEGEN, Nicolas, 8222 Beringen (CH); KAULE, Sebastian, 18057 Rostock (DE); ILLNER, Sabine, 18059 Rostock (DE); KOHSE, Stefanie, 18059 Rostock (DE); GRABOW, Niels, 18055 Rostock (DE); SCHMITZ, Klaus-Peter, 18119 Rostock (DE)
(74) Vertreter: Biotronik Corporate Services SE
(86) Internationale Anmeldenummer: PCT/EP2020/078121
(87) Internationale Veröffentlichungsnummer: WO 2021/073978

(56) Entgegenhaltungen:
- WO-A1-98/51408
- WO-A1-2013/033791
- WO-A1-2013/090145
- WO-A1-2015/055652
- WO-A1-2017/040851
- US-A1- 2016 194 425

## Beschreibung

Die vorliegende Erfindung betrifft ein Dichtungsmaterial für ein medizinisches Implantat gemäß dem Oberbegriff des Anspruchs 1 sowie ein medizinisches Implantat mit einem derartigen Dichtungsmaterial gemäß dem Anspruch 14.

Bei verschiedenen medizinischen Implantaten ergibt sich das Problem, dass nach der Implantation eine Undichtigkeit zwischen der Oberfläche des Implantats und einer anatomischen Struktur des Patienten, beispielsweise einer Gefäßwandung, in der das Implantat implantiert wurde, besteht. Im Fall einer Herzklappenprothese als medizinisches Implantat kann beispielsweise eine paravalvuläre Leckage auftreten, durch die die Leistungsfähigkeit der Herzklappenprothese eingeschränkt wird.

Aus dem Stand der Technik sind bereits zahlreiche Dichtungsmaterialien bekannt, die für medizinische Implantate wie beispielsweise Herzklappenprothesen eingesetzt werden können. Diese Dichtungsmaterialien weisen jedoch Nachteile auf.

Die US 2017/0273786 A1 beschreibt ein medizinisches Implantat mit einem Dichtelement, das beim Überschreiten einer vordefinierten Temperatur expandiert. Um hier eine einheitliche Expansion zu erreichen, muss eine möglichst gleichmäßige Temperaturverteilung innerhalb des Dichtelements realisiert werden. Dies ist regelmäßig nur dann möglich, wenn das Dichtelement eine geringe Wandstärke aufweist, wodurch seine maximal mögliche Dichtfunktion begrenzt wird.

Die US 2013/0190857 A1 beschreibt ein endoluminales Dichtelement zum Abdichten eines endoluminalen Implantats. Dabei expandiert das Dichtelement, wenn es einer Flüssigkeit ausgesetzt wird. Zur Kontrolle des Expansionsprozesses ist dabei der Einsatz einer semipermeablen Membran vorgesehen, durch die hindurch eine Flüssigkeit zum Dichtmaterial gelangen und dessen Expansion auslösen kann.

Die US 2016/0194425 A1 beschreibt stark expandierbare Materialien, die als Dichtmaterialien für medizinische Implantate eingesetzt werden können. Dabei bestehen die Dichtmaterialien aus einem Hydrogel, das in einer Lagerlösung nicht quillt; nach Kontakt mit einem wässrigen Fluid jedoch innerhalb von 15 Minuten auf das 200- bis 1000-fache seines vorherigen Gewichts anschwillt. Dabei ist abermals der Einsatz einer semipermeablen Membran angedacht, durch die hindurch das wässrige Fluid zu dem Hydrogel gelangt.

Die US 2013/0150957 A1 beschreibt ein Gefäßklappensystem, bei dem ein Hydrogel vorgesehen ist, das ein Calcium-chelatierendes Agens sowie ein ansäuerndes Agens aufweist. Dabei wird insbesondere auf die Behandlung kalzifizierter Gefäßklappen abgestellt, nicht jedoch auf eine mögliche Abdichtung durch das Hydrogel.

Die WO 2015/055652 A1 beschreibt eine Dichtung für eine Stent-Prothese, wobei die Dichtung eine Hohlhülse aufweist, die einen Raum für ein erstes Material definiert, welches quillt, wenn es mit einem Bestandteil einer Körperflüssigkeit in Kontakt kommt. Darüber hinaus weist die Dichtung ein zweites Material auf, das zumindest für einige Bestandteile der Körperflüssigkeit durchlässig ist. Mithin beschreibt auch diese internationale Patentanmeldung vorrangig den Einsatz einer semipermeablen Membran zur Aufnahme eines quellfähigen Materials.

Die US 2017/0252155 A1 beschreibt eine Herzklappenprothese mit einem Abschlussmaterial, das beim Kontakt mit Blut die Bildung eines Thrombus induziert, welcher zur Abdichtung der Herzklappenprothese gegenüber einer Gefäßwand eines Patienten dient.

Die WO 2013/033791 A1 beschreibt Dichtungsmaterialien für Gefäßimplantate, enthaltend quellende Hydrogele, die von semipermeablen Membranen umgeben sind.

Der vorliegenden Erfindung liegt die Aufgabe zugrunde, ein für ein medizinisches Implantat geeignetes Dichtungsmaterial bereitzustellen, das die aus dem Stand der Technik bekannten Nachteile überwindet und verbesserte Expansionseigenschaften aufweist.

Diese Aufgabe wird durch ein Dichtungsmaterial für ein medizinisches Implantat mit den Merkmalen des Anspruchs 1 gelöst. Ein solches Dichtungsmaterial weist eine Kompositstruktur mit einer ersten Komponente, einer zweiten Komponente und einer dritten Komponente auf. Die erste Komponente sorgt dabei für eine mechanische Grundstruktur und eine Stabilität der Kompositstruktur. Die erste Komponente weist zu diesem Zweck mindestens ein biologisch inertes Polymer auf. In einer Variante besteht die erste Komponente aus diesem biologisch inerten Polymer. Dieses biologisch inerte Polymer ist typischerweise ein biokompatibles Polymer; also ein Polymer, das selbst bzw. dessen Abbauprodukte nicht toxisch für einen Organismus sind, in dem das Dichtungsmaterial zum Einsatz kommt, und das keine relevanten negativen Einflüsse auf einen solchen Organismus hat.

Die zweite Komponente ist in der Lage, eine große Menge Wasser, die weit über ihre eigene Trockenmasse hinausgeht, aufzunehmen. Dabei weist die zweite Komponente gleichzeitig eine mechanische Stabilität auf. Zu diesem Zweck weist die zweite Komponente ein Hydrogel auf, das nach Kontakt mit einer wässrigen Lösung aufquillt und dadurch innerhalb einer ersten Zeitdauer eine erste Volumenvergrößerung erfährt. Typischerweise geht diese Volumenvergrößerung auch mit einer Massenvergrößerung der zweiten Komponente einher (nämlich durch die Einlagerung von Wasser). In einer Variante besteht die zweite Komponente aus diesem Hydrogel.

Die dritte Komponente ist ebenso in der Lage aufzuquellen. Dabei erfolgt das Aufquellen allerdings innerhalb eines kürzeren Zeitraums als das Aufquellen der zweiten Komponente. Mithin handelt es sich bei der dritten Komponente um eine schnell quellende Substanz. Eine mechanische Stabilität der dritten Komponente ist dabei weniger relevant. Erfindungsgemäß ist vorgesehen, dass die dritte Komponente eine hygroskopische Matrix aufweist, die nach Kontakt mit einer wässrigen Lösung aufquillt und dadurch innerhalb einer zweiten Zeitdauer eine zweite Volumenvergrößerung erfährt. Die schnell quellenden Eigenschaften der dritten Komponente werden dadurch definiert, dass die zweite Zeitdauer kürzer als die erste Zeitdauer ist. In einer Variante besteht die dritte Komponente aus der hygroskopischen Matrix.

Mit dem Kontext der vorliegenden Erfindung umfasst der Begriff "medizinisches Implantat" insbesondere Stent-basierte Implantate und Herzklappenprothesen, insbesondere Aorten-Herzklappenprothesen, welche Stent-basiert sind. Der Begriff "medizinisches Implantat" liest sich erfindungsgemäß auch auf ein jedes medizinisches Implantat, für das das Dichtungsmaterial geeignet ist, das Implantat gegen eine anatomische Struktur abzudichten.

Durch die Kompositstruktur des erfindungsgemäß beanspruchten Dichtungsmaterials ergeben sich gegenüber den aus dem Stand der Technik bekannten Dichtungsmaterialien entscheidende vorteilhafte Effekte: So wird durch die erste Komponente stets eine mechanische Stabilität des Dichtungsmaterials gewährleistet, und zwar unabhängig davon, ob sich das Dichtungsmaterial in seinem nicht-expandierten oder in seinem expandierten (also dichtenden) Zustand befindet. Durch ein Zusammenspiel der zweiten Komponente mit der dritten Komponente wird eine zweistufige Quellung des Dichtungsmaterials erreicht. So gewährleistet die dritte Komponente eine schnelle Quellung, die von einer langsameren Quellung der zweiten Komponente unterstützt wird. Dabei kann durch die schnelle Quellung der dritten Komponente bereits innerhalb der kurzen zweiten Zeitdauer eine Dichtigkeit erreicht werden, die eine beanstandungslose Funktion eines medizinischen Implantats, das mit dem Dichtungsmaterial versehen ist, für einen kurzen Zeitraum gewährleistet. Vor Ablauf dieses kurzen Zeitraums kommt es dann zum Aufquellen der zweiten Komponente, die zudem auch in ihrem aufgequollenen Zustand eine mechanische Stabilität aufweist, die über die mechanische Stabilität der dritten Komponente hinausgeht. Dadurch kann das Dichtungsmaterial eine dauerhafte Dichtigkeit vermitteln und so für eine beanstandungslose Funktion eines medizinischen Implantats, das mit diesem Dichtungsmaterial ausgestattet ist, über einen langen Zeitraum sorgen.

Durch das zweistufige Aufquellen ist somit die schnelle Bereitstellung einer nicht lang anhaltenden Dichtung (nämlich durch die dritte Komponente) und die etwas verzögerte Bereitstellung einer lang anhaltenden Dichtung (nämlich durch die zweite Komponente) gewährleistet. Durch eine derartige, mehrstufig aufgebaute Dichtung wird eine gegenüber den aus dem Stand der Technik bekannten Lösungen verbesserte Dichtungswirkung des vorliegend beanspruchten Dichtungsmaterials erreicht. Dabei dient das Dichtungsmaterial - wenn es zur Abdichtung eines medizinischen Implantats eingesetzt wird - regelmäßig nicht nur zum Abdichten, sondern auch zum Ausgleichen anatomischer Unregelmäßigkeiten und mithin zum Auffüllen ungewollter Lücken / Zwischenräumen zwischen einer anatomischen Struktur und einem medizinischen Implantat, auf dem das Dichtmaterial angeordnet ist.

In einer Variante weist die dritte Komponente eine schwammartige Struktur auf. Durch eine derartige schwammartige Struktur ist es möglich, die dritte Komponente nach einer Expansion wieder zu komprimieren. Dies ist insbesondere dann von Vorteil, wenn das Dichtungsmaterial auf einem medizinischen Implantat eingesetzt wird. Wenn sich während der Implantation herausstellt, dass das Implantat trotz der aufgequollenen dritten Komponente noch nicht richtig gegenüber einer anatomischen Struktur eines Patienten, beispielsweise einer Gefäßwandung, abdichtet oder dass das Implantat nicht wunschgemäß positioniert ist, muss regelmäßig eine Repositionierung des Implantats erfolgen. Zu diesem Zweck wird das Implantat wieder in eine Vorrichtung, die zur Implantation des Implantats verwendet wird, zurückgezogen (sogenanntes "Resheathing"). Für ein derartiges Zurückziehen muss das Implantat aber wieder auf einen sehr viel kleineren Querschnitt komprimiert (gecrimpt) werden als der Querschnitt, in dem es in seinem expandierten Zustand vorliegt. Wenn bereits das gesamte Dichtungsmaterial bzw. ein signifikanter Anteil davon bei den aus dem Stand der Technik bekannten Lösungen expandiert ist, ist ein derartiges Zurückziehen für gewöhnlich nicht mehr möglich. Demgegenüber gestattet gerade die vorliegend beanspruchte Erfindung, insbesondere in der Variante, in der die dritte Komponente eine schwammartige Struktur aufweist, ein solches Zurückziehen / Resheathing, da zunächst nur die dritte Komponente - nicht jedoch die zweite Komponente - expandiert. Das heißt, die Zeitverzögerung, die sich zwischen der Expansion der dritten Komponente und der Expansion der zweiten Komponente ergibt, kann dazu genutzt werden, die dritte Komponente wieder zu komprimieren und das gesamte Implantat zurückzuziehen. Nach einer Repositionierung kann das Implantat wieder in seinen expandierten Zustand überführt werden. Dann kommt es erneut zu einem Aufquellen der dritten Komponente und - wenn das Implantat an der korrigierten Implantationsstelle verbleibt - auch zu einem Aufquellen der zweiten Komponente. Folglich stellt sich eine dauerhaft gute Dichtigkeit zwischen dem medizinischen Implantat und einer anatomischen Struktur des Patienten, beispielsweise einer Gefäßwandung, ein, die durch das erfindungsgemäß beanspruchte Dichtungsmaterial bereitgestellt wird.

In einer Variante weist die erste Komponente oder das mindestens eine biologisch inerte Polymer eine Zugfestigkeit von 0,1 N/mm² bis 20 N/mm², insbesondere von 0,2 N/mm² bis 19 N/mm², insbesondere von 0,5 N/mm² bis 18 N/mm², insbesondere von 1 N/mm² bis 17 N/mm², insbesondere von 2 N/mm² bis 16 N/mm², insbesondere von 3 N/mm² bis 15 N/mm², insbesondere von 4 N/mm² bis 14 N/mm², insbesondere von 5 mm² bis 13 N/mm², insbesondere von 6 N/mm² bis 12 N/mm², insbesondere von 7 N/mm² bis 11 N/mm², insbesondere von 8 N/mm² bis 10 N/mm² auf.

Alternativ oder zusätzlich weist die erste Komponente oder das biologisch inerte Polymer in einer Variante eine Bruchdehnung von 30% bis 500%, insbesondere von 40% bis 450%, insbesondere von 50% bis 400%, insbesondere von 60% bis 350%, insbesondere von 70% bis 300%, insbesondere von 80% bis 250%, insbesondere von 90% bis 200%, insbesondere von 100% bis 150% auf.

In einer Variante weist die zweite Komponente oder das Hydrogel nach der ersten Volumenvergrößerung eine Zugfestigkeit von 0,02 N/mm² bis 1 N/mm², insbesondere von 0,05 N/mm² bis 0,9 N/mm², insbesondere von 0,1 N/mm² bis 0,8 N/mm², insbesondere von 0,2 N/mm² bis 0,7 N/mm², insbesondere von 0,3 N/mm² bis 0,6 N/mm², insbesondere von 0,4 N/mm² bis 0,5 N/mm² auf.

Alternativ oder zusätzlich weist die zweite Komponente oder das Hydrogel in einer Variante eine Bruchdehnung von 30% bis 130%, insbesondere von 40% bis 120%, insbesondere von 50% bis 110%, insbesondere von 60% bis 100%, insbesondere von 70% bis 90% auf.

In einer Variante weist die dritte Komponente oder die hygroskopische Matrix nach der zweiten Volumenvergrößerung eine Zugfestigkeit von bis zu 2,5 N/mm², insbesondere von 0,001 N/mm² bis 2,5 N/mm², insbesondere von 0,01 N/mm² bis 2 N/mm², insbesondere von 0,1 N/mm² bis 1,5 N/mm², insbesondere von 0,5 N/mm² bis 1 N/mm² auf.

Alternativ oder zusätzlich weist die dritte Komponente oder die hygroskopische Matrix in einer Variante eine Bruchdehnung von 10% bis 40%, insbesondere von 15% bis 35%, insbesondere von 20% bis 30% auf.

Dabei wird das Material für die dritte Komponente bzw. die hygroskopische Matrix typischerweise derart gewählt, dass es eine geringere mechanische Stabilität als das Material der zweiten Komponente bzw. des Hydrogels aufweist.

In allen der vorgenannten alternativen Ausgestaltungen werden die Zugfestigkeit und die Bruchdehnung gemäß ISO 527 bestimmt.

In einer Variante beträgt die erste Zeitdauer 1 Stunde bis 10 Stunden, insbesondere 1,5 Stunden bis 9,5 Stunden, insbesondere 2 Stunden bis 9 Stunden, insbesondere 2,5 Stunden bis 8,5 Stunden, insbesondere 3 Stunden bis 8 Stunden, insbesondere 3,5 Stunden bis 7,5 Stunden, insbesondere 4 Stunden bis 7 Stunden, insbesondere 4,5 Stunden bis 6,5 Stunden, insbesondere 5 Stunden bis 6 Stunden.

Alternativ oder zusätzlich hat die erste Volumenvergrößerung in einer Variante eine Vergrößerung eines initialen Volumen des Hydrogels um mindestens einen Faktor 2 zur Folge. Die erste Volumenvergrößerung bewirkt insbesondere eine Vergrößerung des initialen Volumens des Hydrogels um einen Faktor von 2 bis 1000, insbesondere von 5 bis 900, insbesondere von 10 bis 800, insbesondere von 20 bis 700, insbesondere von 30 bis 600, insbesondere von 40 bis 500, insbesondere von 50 bis 400, insbesondere von 60 bis 300, insbesondere von 70 bis 200, insbesondere von 80 bis 100. Dabei sind sämtliche der vorstehend genannten Zeitdauern mit sämtlichen der vorstehend genannten Volumenvergrößerungen miteinander kombinierbar.

Eine erste Zeitdauer von 5 Stunden bis 7 Stunden ist besonders geeignet. Darüber hinaus ist eine Volumenvergrößerung um einen Faktor von 20 bis 60 besonders geeignet, wobei eine Kombination der vorstehend genannten besonders geeigneten Intervalle ebenfalls besonders geeignet ist.

In einer Variante beträgt die zweite Zeitdauer 10 Sekunden bis 59 Minuten, insbesondere 15 Sekunden bis 50 Minuten, insbesondere 20 Sekunden bis 40 Minuten, insbesondere 30 Sekunden bis 30 Minuten, insbesondere 40 Sekunden bis 20 Minuten, insbesondere 50 Sekunden bis 10 Minuten, insbesondere 60 Sekunden bis 5 Minuten, insbesondere 90 Sekunden bis 4 Minuten, insbesondere 2 Minuten bis 3 Minuten. Eine besonders geeignete zweite Zeitdauer beträgt 60 Sekunden bis 120 Sekunden.

Alternativ oder zusätzlich hat die zweite Volumenvergrößerung eine Vergrößerung eines initialen Volumens der hygroskopischen Matrix um mindestens einen Faktor 2 zur Folge. Insbesondere bewirkt die zweite Volumenvergrößerung eine Vergrößerung des initialen Volumens der hygroskopischen Matrix um einen Faktor von 2 bis 1000, insbesondere von 5 bis 900, insbesondere von 10 bis 800, insbesondere von 20 bis 700, insbesondere von 30 bis 600, insbesondere von 40 bis 500, insbesondere von 50 bis 400, insbesondere von 60 bis 300, insbesondere von 70 bis 200, insbesondere von 80 bis 100. Dabei sind sämtliche der vorstehend genannten Zeitdauern mit sämtlichen der vorstehend genannten Volumenvergrößerungen miteinander kombinierbar.

Mit dem Kontext der Erfindung sei erwähnt, dass das Quellverhalten einer hygroskopischen Matrix typischerweise von deren Dicke / Stärke abhängig ist, in der es verwendet wird. Je dicker / stärker diese Schicht ist, desto mehr Wasser kann prinzipiell gebunden werden.

In einer Variante quillt das Hydrogel der zweiten Komponente nicht nur durch einen Kontakt mit einer wässrigen Flüssigkeit. Vielmehr ist es in dieser Variante derart beschaffen, dass ein externer Stimulus dafür sorgt, dass die erste Volumenvergrößerung beschleunigt oder vergrößert (verstärkt) wird. Dieser externe Stimulus kann beispielsweise eine gegenüber einem Ausgangswert erhöhte Temperatur, ein gegenüber einem Ausgangswert erhöhter oder erniedrigter pH-Wert und/oder eine gegenüber einer Ausgangskonzentration erhöhte oder erniedrigte Ionenkonzentration sein. Wenn das Hydrogel auf einen derartigen externen Stimulus anspricht, können die Quelleigenschaften des Hydrogels zusätzlich beeinflusst werden.

In einer Variante ist das mindestens eine biologisch inerte Polymer ausgewählt aus der Gruppe bestehend aus Polyurethanen, Polyimiden, Polyethylenen, Polypropylenen, Polysulfonen, Polyestern, Polytetrafluoroethylen, Silikonen, Fluorosilikonen, Polyaryletherketonen, Polyvinylidenfluorid, Vinylpyrrolidon/Vinylacetat-Copolymeren und Polyvinylfluorid.

In einer Variante weist das Hydrogel mindestens eine Substanz auf, die ausgewählt ist aus der Gruppe bestehend aus polymerisierbaren ionischen Flüssigkeiten (II,s), thermosensitiven Polymeren, Polyacrylamiden, Polyoxazolinen, Polyvinylethern und Polyethylenglykolen. Vinyloge Imidazolium-basierte ionischen Flüssigkeiten wie beispielsweise 1-Vinyl-3-isopropylimidazoliumbromid sind besonders geeignete ionische Flüssigkeiten.

In einer Variante weist die hygroskopische Matrix mindestens eine Substanz auf, die ausgewählt ist aus der Gruppe bestehend aus Zellstoffmatrix, Cellulose-Derivaten und Chitosan.

In einer Variante weist die zweite Komponente mindestens eine Substanz zur Förderung der Endothelialisierung auf. Hierbei kann es sich beispielsweise um einen Endothelwachstumsfaktor (VEGF) handeln. Wenn ein derartiger Wachstumsfaktor in die zweite Komponente bzw. das Hydrogel integriert wird, wird durch die starke Quellung des Hydrogels eine sehr starke Diffusion des Wachstumsfaktors in die Umgebung der zweiten Komponente erreicht. Wenn das Dichtungsmaterial beispielsweise auf einer Herzklappenprothese als medizinischem Implantat angeordnet ist, ist es somit möglich, eine starke Diffusion eines Wachstumsfaktors in ein kardiales Gewebe wie beispielsweise einen aortalen Annulus oder einen mitralen Annulus zu erreichen. Dadurch kann das Endothelwachstum in diesen Geweben verstärkt werden, was zu einem verbesserten Einwachsen der mit dem Dichtungsmaterial versehenen Herzklappenprothese führt. Durch den beschleunigten Integrationsprozess wird der Heilungsprozess des betreffenden Patienten beschleunigt, was in einer insgesamt besseren Verträglichkeit einer entsprechend ausgerüsteten Herzklappenprothese und einer größeren Akzeptanz einer solchen Herzklappenprothese gegenüber den aus dem Stand der Technik bekannten Prothesen resultiert.

In einer Variante weist die hygroskopische Matrix mindestens eine Substanz mit antikalzifizierender Wirkung auf. Hierbei kann es sich beispielsweise um einen Härtestabilisator handeln. Substanzen mit einer antikalzifizierenden Wirkung können eine Hemmung einer Kalzifizierung unterstützen oder sogar eine allmähliche Rückbildung bereits bestehender Kalkablagerungen bewirken. Ein Beispiel für eine derartige Substanz mit antikalzifizierender Wirkung ist Polysuccinimid. Bereits bei leicht erhöhtem pH-Wert wird Polysuccinimid partiell hydrolysiert und dadurch in hochvernetzter Form quellungsfähig bzw. in linearer Form wasserlöslich. Dadurch entsteht Polyasparaginsäure, die sich als langanhaltend wirksamer Inhibitor anorganischer Kalkablagerungen eignet. Durch die Einbringung einer derartigen Substanz mit antikalzifizierender Wirkung in die hygroskopische Matrix, bzw. in die dritte Komponente, wird die Gesamtstabilität der Kompositstruktur des Dichtungsmaterials nicht beeinflusst, da die dritte Komponente nicht wesentlich zur Stabilität der Kompositstruktur beiträgt. Wenn einer Neubildung von Kalkablagerungen effektiv entgegengewirkt werden kann, bzw. wenn bestehende Kalkablagerungen abgebaut werden können, erhöht sich die Dauerfestigkeit eines Implantats, das mit dem erfindungsgemäß beanspruchten Dichtungsmaterial ausgestattet ist. Dadurch können Revisionsoperationen oder Valve-in-valve-Operationen vermieden werden. Als Folge steigt die Akzeptanz derart ausgestatteter Implantate bei betroffenen Patienten und Operateuren.

In einer Variante weist das Dichtungsmaterial einen Schichtaufbau auf. Dabei bildet die erste Komponente einen Kernbereich des Dichtungsmaterials. Die zweite Komponente bildet eine erste Schicht, die den Kernbereich umgibt. Die dritte Komponente bildet eine zweite Schicht, die die erste Schicht umgibt. Mithin kommt die dritte, schnell quellende Komponente beim Einsatz des Dichtungsmaterials zuerst in Kontakt mit einer wässrigen Lösung und kann durch ihr schnelles Aufquellen schnell eine erste Dichtungswirkung des Dichtungsmaterials vermitteln. Nachfolgend kommt die zweite, langsamer quellende Komponente in Kontakt mit der wässrigen Lösung und kann eine länger anhaltende und stabilere Dichtungswirkung aufbauen. Ob die erste Komponente in Kontakt mit einer wässrigen Lösung kommt ist unerheblich, da sie ihre strukturellen Eigenschaften unabhängig davon behält, ob sie in Kontakt mit einer wässrigen Lösung steht oder nicht. Durch diesen mehrschichtigen Aufbau können die vorteilhaften Eigenschaften des Dichtungsmaterials in besonders geeigneter Art und Weise realisiert werden.

Die erste Komponente kann in einem derartigen mehrschichtigen Aufbau beispielsweise als Monofilament vorliegen; also in Form einer kompakten Ausgestaltung. Es ist alternativ auch möglich, dass die erste Komponente als Multifilament vorliegt, also als Ansammlung zahlreicher beieinanderliegender Monofilamente. Dann ist die erste Komponente typischerweise weniger kompakt ausgebildet als im Fall einer monofilen ersten Komponente.

In einer alternativen Ausgestaltung weist das Dichtungsmaterial keinen mehrschichtigen Aufbau auf. Vielmehr weist es in dieser Variante einen multifilen Aufbau auf, der aus einer Vielzahl (zwei oder mehr) Filamente der ersten Komponente, einer Vielzahl Filamente der zweiten Komponente und einer Vielzahl Filamente der dritten Komponente besteht, die regelmäßig oder unregelmäßig zueinander angeordnet sind. Ein derartiger Aufbau kann auch als Kompositfaden bezeichnet werden.

In einer Variante liegen die zweite Komponente bzw. das Hydrogel und die dritte Komponente bzw. die hygroskopische Matrix als Kompositmaterial vor. In dieser Variante ist es also vorgesehen, die zweite Komponente und die dritte Komponente derart miteinander zu vermengen, dass sie für den makroskopischen Aufbau des Dichtungsmaterials letztlich wie eine einzelne Komponente wirken. Dieses Kompositmaterial aus zweiter Komponente und dritter Komponente weist dann ein zweistufiges Quellverhalten auf, das heißt, es vereint die oben beschriebenen unterschiedlichen Quellungseigenschaften der zweiten Komponente und der dritten Komponente in sich. Der Aufbau makroskopischer Strukturen aus einem derartigen Kompositmaterial kann gegenüber dem Einsatz dreier getrennter Komponenten vereinfacht werden. Allerdings sind die Quellungseigenschaften der letztlich eingesetzten Kompositstruktur beim Einsatz eines Kompositmaterials aus zweiter Komponente und dritter Komponente schlechter beeinflussbar bzw. steuerbar.

Ein derartiges Kompositmaterial kann insbesondere dann besonders einfach hergestellt werden, wenn eine polymerisierbare ionische Flüssigkeit als zweite Komponente oder in der zweiten Komponente eingesetzt wird und Cellulosederivate als dritte Komponente oder in der dritten Komponente eingesetzt werden. Denn Cellulosederivate sind in polymerisierbaren ionischen Flüssigkeiten gut löslich, sodass sich Kompositmaterialien besonders einfach herstellen lassen. Dadurch werden Diffusionsbarrieren abgebaut und die Quellzeit insgesamt weiter verringert. Fasern oder Fäden aus einem derartigen Kompositmaterial lassen sich in den vorstehend oder nachfolgend erläuterten Varianten wie Fasern oder Fäden aus einer einzigen Komponente einsetzen.

In einer Variante liegt das Dichtungsmaterial in Form eines monofilen Fadens, in Form eines multifilen Fadens, in Form eines Tauwerks, in Form eines Gestricks, in Form eines Gewirks, in Form einer Flechtung und/oder in Form eines Vlieses vor.

Wie bereits oben erwähnt, ist im Fall eines monofilen Fadens insbesondere die erste Komponente als monofiler Polymerfaden ausgestaltet, während die zweite Komponente und die dritte Komponente insbesondere als Beschichtung um diesen monofilen Polymerfaden herum angeordnet sind. Dabei kann die erste Komponente beispielsweise als Faser mit einem Durchmesser im Mikrometerbereich bis Millimeterbereich ausgestaltet werden und nachfolgend schrittweise mit der zweiten Komponente und der dritten Komponente beschichtet werden. Hierzu eignen sich beispielsweise Tauch-, Sprüh- oder Plasmapolymerisationsverfahren.

Im Fall eines multifilen Fadens ergeben sich verschiedene Ausgestaltungsmöglichkeiten der Kompositstruktur. Beispielsweise kann lediglich die erste Komponente in Form eines multifilen Polymerfadens ausgestaltet und abermals - wie ebenfalls oben bereits erläutert - durch die zweite Komponente und die dritte Komponente beschichtet sein. Ein derartiger multifiler Polymerfaden kann beispielsweise aus einer (elektro-)gesponnenen oder extrudierten ersten Komponente hergestellt werden. Man kann die erste Komponente dann auch als Kern aus versponnenen Stapelfasern bezeichnen. Anschließend erfolgt eine schrittweise Beschichtung mit der zweiten oder dritten Komponente. Auch hierfür eignen sich beispielsweise Tauch-, Sprüh-, oder Plasmapolymerisationsverfahren.

Es ist aber auch möglich, dass die einzelnen Komponenten für sich genommen in Form von Filamenten vorliegen, die zusammen in regelmäßiger oder unregelmäßiger Anordnung einen multifilen Faden ergeben. Ein derartiger multifiler Faden kann beispielsweise aus einzeln oder zusammen (elektro-)gesponnenen oder extrudierten, gerichteten Polymerfasern hergestellt werden. Hierfür können beispielsweise drei separate Düsen verwendet werden, um die drei unterschiedlichen Komponenten gleichzeitig zu spinnen bzw. zu extrudieren. In einem weiteren Prozessschritt werden dann die drei derart gesponnenen oder extrudierten Komponenten zu einem multifilen Faden (der auch als Garn bezeichnet werden kann) gebündelt / verzwirnt.

Ein weiterer multifiler Faden kann beispielsweise aus mehreren Drei-Komponenten-Fasern hergestellt werden, beispielsweise durch triaxial elektrogesponnene, gerichtete Polymerfasern. Diese können über eine einzige, triaxial aufgebaute Düse elektrogesponnen werden und in einem nachfolgenden Verfahrensschritt zu einem multifilen Faden (der auch als Garn bezeichnet werden kann) gebündelt / verzwirnt werden.

Ein Tauwerk ist eine aus mehreren Fäden bestehende Struktur, bei der die einzelnen Fäden umeinander greifen. Eine Ausführung des Dichtungsmaterials als Tauwerk ermöglicht es, die Eigenschaften der einzelnen Komponenten ortsaufgelöst einzusetzen. Dies ist insbesondere dann von besonderer Bedeutung, wenn das Dichtungsmaterial als Teil eines medizinischen Implantats, insbesondere zur Abdichtung eines medizinischen Implantats gegenüber einem mitralen Annulus eingesetzt werden soll. Denn dann können durch eine entsprechende Ortsauflösung besonders vorteilhafte Dichtungseigenschaften des Dichtungsmaterials erreicht werden.

Ein Tauwerk kann als verzwirntes / gedrehtes Tauwerk realisiert werden. Ein derartiges Tauwerk besteht aus monofilen Strängen der ersten Komponente, der zweiten Komponente und der dritten Komponente und kann beispielsweise durch Extrusion oder umformende Fertigungsverfahren hergestellt werden. Gleichermaßen können für ein derartiges verzwirntes oder gedrehtes Tau auch monofile Kompositfäden eingesetzt werden, bei der die drei Einzelkomponenten wie beim oben beschriebenen monofilen Faden angeordnet sind (ein monofiler Kern aus der ersten Komponente mit einer Beschichtung aus der zweiten Komponente und der dritten Komponente).

Ein Tau kann schließlich auch aus multifilen Polymerfäden hergestellt werden, wie diese vorstehend bereits erläutert wurden.

Ein Strick, ein Gewirk oder eine Flechtung kann aus den vorstehend erläuterten Fäden, dem vorstehend erläuterten Tauwerk oder Kombinationen aus den vorstehend erläuterten Fäden und dem vorstehend erläuterten Tauwerk hergestellt werden.

Ein Vlies, das auch als nicht gewebte flächige Struktur bezeichnet werden kann, weist eine Vielzahl ungerichteter oder gerichteter Polymerfasern, insbesondere eine Vielzahl monofiler, dreikomponentiger Polymerfasern auf. Beispielsweise ist es möglich, mehrere Schichten gerichteter Polymerfasern übereinander anzuordnen, um eine derartige Vliesstruktur herzustellen.

In einer Variante weist ein derartiges Vlies eine Porosität zwischen 2 µm und 20 µm, insbesondere zwischen 3 µm und 19 µm, insbesondere zwischen 4 µm und 18 µm, insbesondere zwischen 5 µm und 17 µm, insbesondere zwischen 5 µm und 16 µm, insbesondere zwischen 6 µm und 15 µm, insbesondere zwischen 7 µm und 14 µm, insbesondere zwischen 8 µm und 13 µm, insbesondere zwischen 9 µm und 12 µm, insbesondere zwischen 11 µm und 12 µm auf.

Die Kombination von drei Komponenten zu einer Kompositstruktur eröffnet neue Möglichkeiten für ein schnelles, steuerbares und starkes Quellverhalten bei gleichzeitig verbesserter mechanischer Stabilität der gesamten Struktur. Dabei ist es in einer Variante möglich, das Quellverhalten durch externe Stimuli sowie durch die Anordnung der einzelnen Komponenten des Kompositmaterials gezielt zu regulieren bzw. anzupassen. Gleichermaßen lässt sich die mögliche Abgabe von zusätzlich in den Einzelkomponenten enthaltenen Wirkstoffen gezielter steuern. Da die Kompositstruktur in Form von Fäden, Tauwerken, aus Fäden und/oder Tauwerk hergestellten größeren Strukturen und/oder aus Vliesen aufgebaut werden kann, ergibt sich ein breiteres Anwendungsfeld als dies bei den aus dem Stand der Technik bekannten Dichtungsmaterialien der Fall ist. Darüber hinaus sind texturabhängige Eigenschaften wie Stärke, Dehnbarkeit, Orientierung des Dichtungsmaterials etc. besser modulierbar und an die jeweiligen Anforderungen anpassbar.

Zusammengefasst stellt das erfindungsgemäße Dichtungsmaterial eine Kompositstruktur dar, die
- in verschiedenen Aufbauten (beispielsweise Fäden/Tauwerk/Gewirk/Flächenstruktur) eingesetzt werden kann;
- die nach klassischen textilen Verarbeitungstechniken zu speziell konditionierten und konfektionierten Geweben verarbeitet werden kann;
- die gerichtet quellen kann;
- die permanent im Körper eines Patienten verbleiben kann;
- die eine retardierte Abgabe von Wirkstoffen ermöglicht, welche in einzelne Komponenten der Kompositstruktur eingebettet sind;
- die zur Förderung der Endothelialisierung einen Wachstumsfaktor abgeben kann;
- die eine insbesondere lang anhaltende antikalzifizierende Wirkung durch den Zusatz entsprechender Substanzen wie Härtestabilisatoren vermitteln kann;
- die sich an einer Herzklappenprothese, insbesondere an der Stentstruktur einer Herzklappenprothese, befestigen lässt, und
- die sich zur Abdichtung zwischen einer Herzklappenprothese, wie etwa einer Transkatheter-Aortenklappenprothese (TAVI, TAVR, PAVR), und einer Gefäßwandung eines Patienten, wie beispielsweise einem aortalen Annulus, eignet.

Ein Aspekt der vorliegenden Erfindung betrifft ein medizinisches Implantat, das ein Dichtungsmaterial gemäß den vorstehenden Erläuterungen aufweist. Dabei ist dieses Dichtungsmaterial an zumindest einem Bereich der Oberfläche des Implantats angeordnet. Bei diesem Bereich handelt es sich um einen Bereich, der im implantierten Zustand des Implantats dafür vorgesehen und eingerichtet ist, ein Gefäß eines Patienten, dem das medizinische Implantat implantiert wurde, zu kontaktieren. Auf diese Weise ist es möglich, dass das Dichtungsmaterial seine dichtenden Eigenschaften zwischen dem implantierten medizinischen Implantat und einer anatomischen Struktur eines Patienten, beispielsweise einer Gefäßwandung, wo das Implantat implantiert wurde, auszuüben.

In einer Variante handelt es sich bei dem medizinischen Implantat um eine Gefäßklappenprothese, insbesondere um eine Herzklappenprothese. Dabei sind beispielsweise eine Aortenklappenprothese, eine Trikuspidalklappenprothese und eine Mitralklappenprothese geeignete Beispiele für eine Herzklappenprothese. Typischerweise weisen derartige Prothesen bzw. Implantate eine stentartige Struktur auf, die in ihrem Inneren eine Ventilanordnung trägt, die eine natürliche Gefäß- oder Herzklappe ersetzt. Dabei kann das Dichtungsmaterial auf die Oberfläche der Herzklappenprothese aufgebracht sein oder beispielsweise in Form eines Fadens oder eines Tauwerks um einzelne Abschnitte der Stentstruktur des medizinischen Implantats herumgewickelt sein.

In einer Variante handelt es sich bei dem medizinischen Implantat um ein trocken gelagertes und/oder trocken ausgeliefertes Gesamtsystem, insbesondere um eine trocken gelagerte / trocken ausgelieferte Herzklappenprothese. Denn sobald das Dichtungsmaterial in Kontakt mit Wasser bzw. einer wässrigen Flüssigkeit wie Blut gelangt, beginnt es zu quellen. Folglich wird das Dichtungsmaterial sinnvollerweise in einem dehydrierten Zustand gelagert und für die Operation bereitgestellt, damit es erst im Körper eines Patienten in Kontakt mit Blut kommt, wodurch dann ein Aufquellen der dritten Komponente und der zweiten Komponente initiiert wird.

In einer Variante wird die Herzklappenprothese zusammen mit dem Dichtungsmaterial in einem dehydrierten Zustand in ein sogenanntes Katheter-Delivery-System geladen und wird in diesem vorgeladenen Zustand in einen Operationssaal geliefert.

Sämtliche Varianten des Dichtungsmaterials können in beliebiger Weise miteinander kombiniert werden und können in jeder beliebigen Kombination auf das beschriebene medizinische Implantat übertragen werden, und umgekehrt.

Weitere Details von Aspekten der vorliegenden Erfindung werden nachfolgend anhand von Ausführungsbeispielen und Figuren näher erläutert. Es zeigen:
- Fig. 1: ein erstes Ausführungsbeispiel eines Fadens aus einem Dichtungsmaterial;
- Fig. 2: ein zweites Ausführungsbeispiel eines Fadens aus einem Dichtungsmaterial;
- Fig. 3: ein drittes Ausführungsbeispiel eines Fadens aus einem Dichtungsmaterial;
- Fig. 4: ein viertes Ausführungsbeispiel eines Fadens aus einem Dichtungsmaterial;
- Fig. 5: eine beispielhafte Darstellung eines Tauwerks aus einem Dichtungsmaterial;
- Fig. 6: ein erstes Ausführungsbeispiel des Aufbaus eines Tauwerks aus einem Dichtungsmaterial;
- Fig. 7: ein zweites Ausführungsbeispiel des Aufbaus eines Tauwerks aus einem Dichtungsmaterial;
- Fig. 8: ein drittes Ausführungsbeispiel des Aufbaus eines Tauwerks aus einem Dichtungsmaterial;
- Fig. 9A: ein Ausführungsbeispiel eines Gestricks aus einem Dichtungsmaterial;
- Fig. 9B: ein Ausführungsbeispiel eines Gewirks aus einem Dichtungsmaterial;
- Fig. 9C: ein Ausführungsbeispiel einer Flechtung aus einem Dichtungsmaterial;
- Fig. 10: ein Ausführungsbeispiel eines Vlieses aus einem Dichtungsmaterial;
- Fig. 11A: eine schematische Darstellung eines ersten Ausführungsbeispiels einer Herzklappenprothese mit einem Dichtungsmaterial;
- Fig. 11B: ein zweites Ausführungsbeispiel einer Herzklappenprothese mit einem Dichtungsmaterial;
- Fig. 11C: ein drittes Ausführungsbeispiel einer Herzklappenprothese mit einem Dichtungsmaterial;
- Fig. 12A: eine erste schematische Querschnittsdarstellung einer Aorta mit implantierter Herzklappenprothese;
- Fig. 12B: eine zweite schematische Querschnittsdarstellung einer Aorta mit implantierter Herzklappenprothese;
- Fig. 12C: eine dritte schematische Querschnittsdarstellung einer Aorta mit implantierter Herzklappenprothese;
- Fig. 13A: ein viertes Ausführungsbeispiel einer Herzklappenprothese mit einem Dichtungsmaterial;
- Fig. 13B: ein fünftes Ausführungsbeispiel einer Herzklappenprothese mit einem Dichtungsmaterial;
- Fig. 13C: eine schematische Ansicht eines mitralen Annulus mit implantierter Herzklappenprothese, und
- Fig. 14: eine schematische Darstellung des Funktionsprinzips der Straffung von Klappensegeln mithilfe eines Dichtungsmaterials.

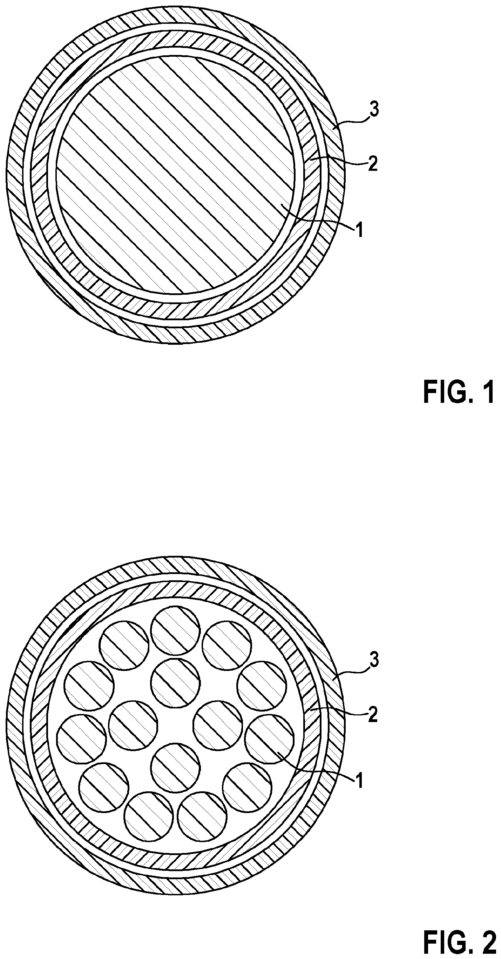

Die Figur 1 zeigt einen monofilen Faden aus einem Dichtungsmaterial, das eine erste Komponente 1, eine zweite Komponente 2 und eine dritte Komponente 3 aufweist. Die erste Komponente 1 weist ein biologisch inertes Polymer auf, das zu einer einzelnen Polymerfaser gesponnen ist. Der Faserdurchmesser liegt typischerweise im Mikrometerbis Millimeterbereich. Die zweite Komponente 2 ist mittels Tauch-, Sprüh-, oder Plasmapolymerisationsverfahren auf die erste Komponente 1 aufgetragen. In gleicher Weise ist die dritte Komponente mittels Tauch-, Sprüh- oder Plasmapolymerisationsverfahren auf die zweite Komponente 2 aufgetragen. Dadurch ergibt sich ein schichtartiger Aufbau, bei dem die erste Komponente 1 den Kern und die zweite Komponente 2 sowie die dritte Komponente 3 eine Beschichtung darstellen.

Die zweite Komponente 2 weist dabei ein Hydrogel auf, das nach Kontakt mit einer wässrigen Lösung aufquillt. Die dritte Komponente 3 weist eine hygroskopische Matrix auf und quillt nach einem Kontakt mit einer wässrigen Lösung ebenfalls auf, allerdings deutlich schneller als die zweite Komponente 2. Somit weist der monofile Polymerfaden der Figur 1 zeitlich gestaffelte Quellungseigenschaften auf.

Die Figur 2 zeigt einen multifilen Polymerfaden aus der ersten Komponente 1, der zweiten Komponente 2 und der dritten Komponente 3. Dabei werden in dieser und in allen folgenden Figuren gleiche Elemente mit jeweils gleichen Bezugszeichen versehen. Darüber hinaus weisen die erste Komponente 1, die zweite Komponente 2 und die dritte Komponente 3 in allen Ausführungsbeispielen die im Zusammenhang mit der Figur 1 erläuterte Zusammensetzung auf, sofern nicht explizit etwas anderes angegeben wird.

Die erste Komponente 1 ist beim Ausführungsbeispiel der Figur 2 ein elektrogesponnener oder extrudierter Faden, der aus einzelnen Fasern der ersten Komponente 1 besteht. Dieser aus einzelnen Fasern bestehende Faden aus der ersten Komponente 1 wird dann schrittweise mit der zweiten Komponente 2 und der dritten Komponente 3 beschichtet. Dies kann abermals beispielsweise mittels einem Tauch-, Sprüh- oder Plasmapolymerisationsverfahren erfolgen.

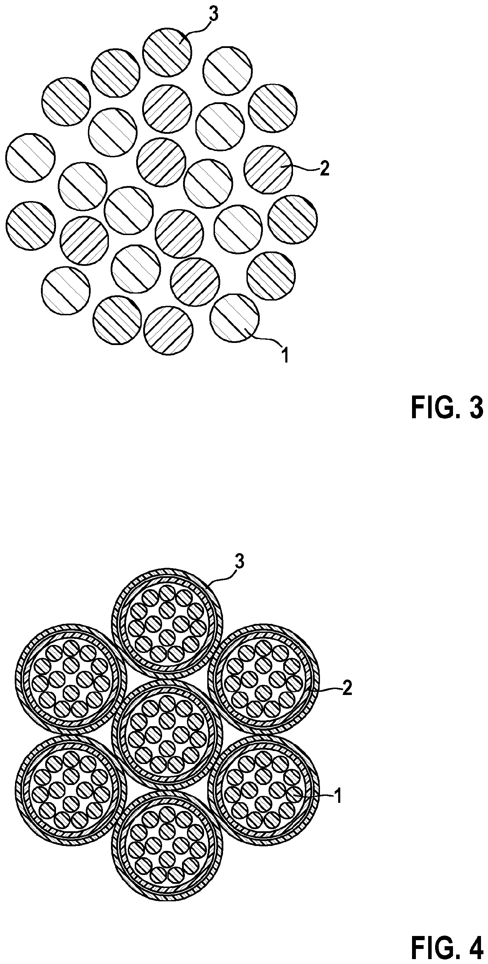

Die Figur 3 zeigt ebenfalls den Querschnitt eines multifilen Polymerfadens. Dabei sind jedoch einzelne Fasern der ersten Komponente 1, der zweiten Komponente 2 und der dritten Komponente 3 unregelmäßig zueinander angeordnet. Ein derartiger multifiler Faden kann aus einzeln oder zusammen gesponnenen oder extrudierten gerichteten Polymerfasern hergestellt werden, indem die Fasern aus der ersten Komponente 1, die Fasern aus der zweiten Komponente 2 und die Fasern aus der dritten Komponente 3 in einem weiteren Verfahrensschritt gebündelt bzw. verzwirnt werden.

Die Figur 4 zeigt ein weiteres Ausführungsbeispiel eines multifilen Fadens, bei dem einzelne Drei-Komponenten-Fäden, die jeweils aus der ersten Komponente 1, der zweiten Komponente 2 und der dritten Komponente 3 bestehen, zu einem multifilen Faden gebündelt bzw. verzwirnt werden. Die Drei-Komponenten-Fäden lassen sich über eine einzige, triaxial aufgebaute Düse elektrospinnen.

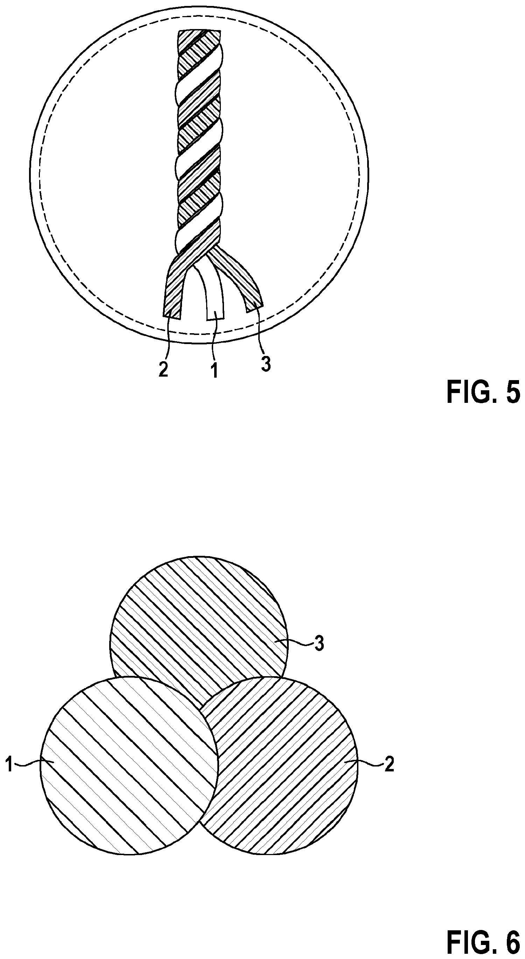

Die Figur 5 zeigt eine schematische Ansicht eines Tauwerks, das aus einzelnen Strängen der ersten Komponente 1, der zweiten Komponente 2 und der dritten Komponente 3 besteht. In einem derartigen Tauwerk können die Eigenschaften der einzelnen Komponenten ortsaufgelöst arrangiert werden. In den Figuren 6 bis 8 sind mögliche Aufbauten eines derartigen Tauwerks näher beschrieben.

So zeigt die Figur 6 einen Querschnitt durch ein Tauwerk, bei dem einzelne Stränge der ersten Komponente 1, der zweiten Komponente 2 und der dritten Komponente 3 miteinander verzwirnt bzw. gegeneinander verdreht sind. Ein derartiges Tau kann beispielsweise durch Extrusion oder umformende Fertigungsverfahren der einzelnen Stränge hergestellt werden.

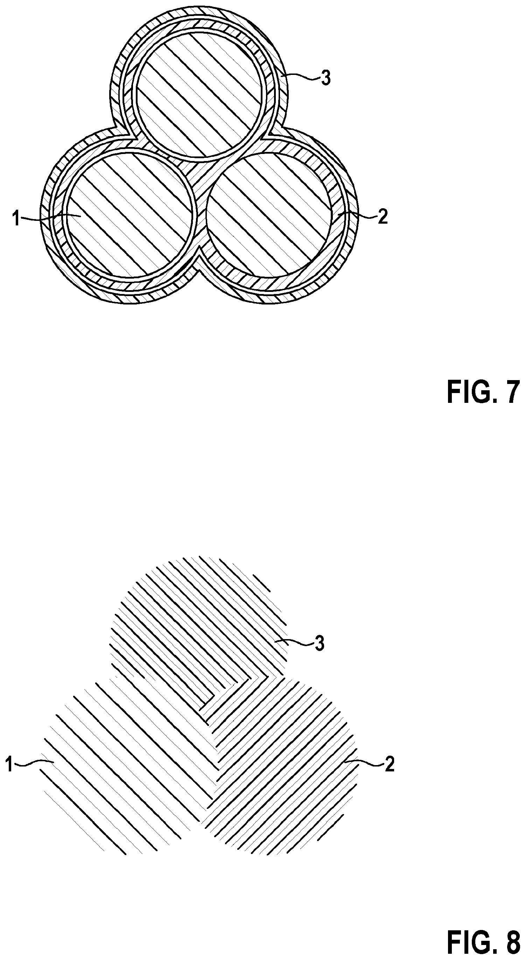

Die Figur 7 zeigt ein Tau, das aus monofilen Kompositfäden besteht. Jeder dieser Kompositfäden weist die erste Komponente 1, die zweite Komponente 2 und die dritte Komponente 3 auf. Der Aufbau eines einzelnen solchen Kompositfadens ist in der Figur 1 näher beschrieben. Auch diese einzelnen Kompositfäden sind miteinander verzwirnt bzw. zueinander verdreht.

Die Figur 8 zeigt den Querschnitt durch ein Tau aus multifilen Fäden, die grundsätzlich wie die in den Figuren 2, 3 und 4 gezeigten Fäden aufgebaut sein können. Dabei sind die einzelnen für das Tau der Figur 8 verwendeten Fäden monokomponentige multifile Fäden; entsprechen also von ihrem Aufbau her den einzelnen Strängen des Fadens des Ausführungsbeispiels der Figur 3.

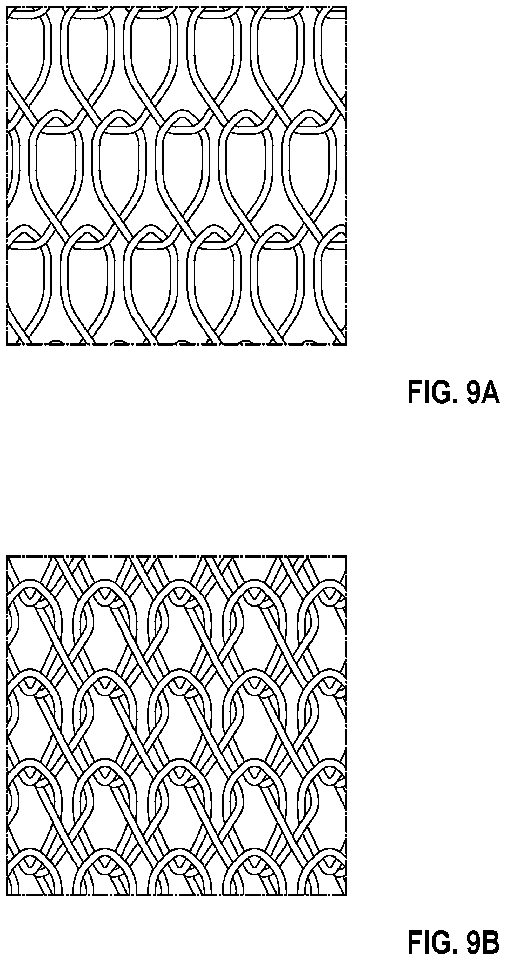

Aus den unterschiedlichen Fäden der Figuren 1 bis 4 oder den unterschiedlichen Tauwerken der Figuren 5 bis 8 lassen sich flächige Gebilde erzeugen, mit denen größere Bereiche mit dem erfindungsgemäß beanspruchten Dichtungsmaterial abgedeckt werden können. Die Figur 9A zeigt ein erstes Ausführungsbeispiel eines solchen flächigen Gebildes, nämlich in Form eines Stricks (mitunter auch als Gestrick bezeichnet). Zur Herstellung eines solchen Stricks werden einzelne Fäden oder einzelne Tauwerke bzw. Kombinationen von Fäden und Tauwerken miteinander verstrickt.

Die Figur 9B zeigt ein flächiges Gebilde in Form eines Gewirks. Auch hier können einzelne Fäden, einzelne Tauwerke oder Kombinationen aus Fäden und Tauwerken miteinander gewirkt werden.

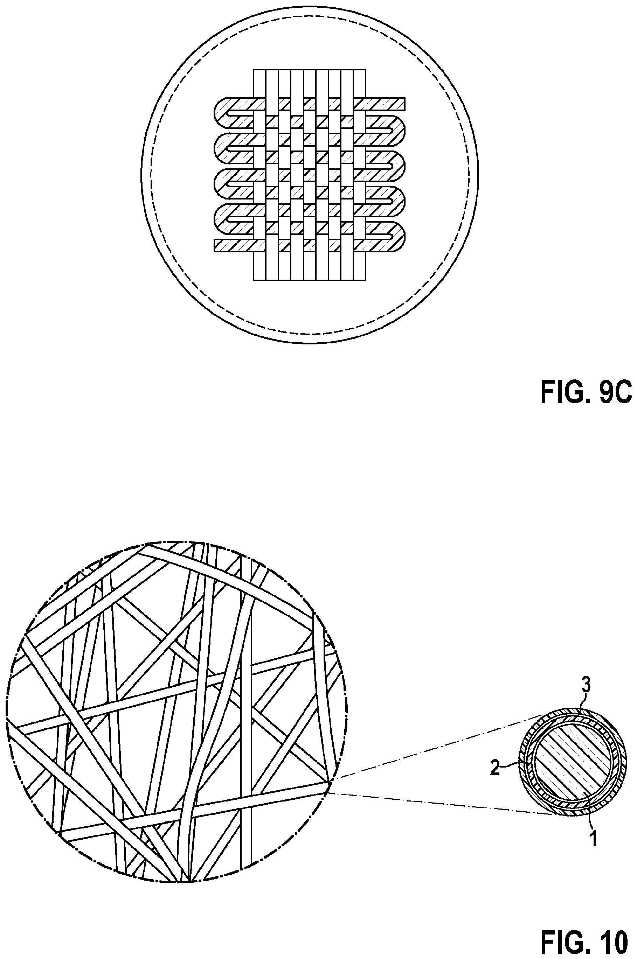

Die Figur 9C zeigt schließlich eine Flechtung (mitunter auch als Gewebe bezeichnet) aus einzelnen Fäden oder einzelner Tauwerke bzw. aus eine Kombination aus Fäden und Tauwerk. Mithin lassen sich die Fäden und das Tauwerk aus dem Dichtungsmaterial nach klassischen textilen Verarbeitungstechniken zu verschiedenen Geweben verarbeiten.

Die Figur 10 zeigt eine weitere mögliche Flächenstruktur des Dichtungsmaterials, nämlich in Form eines Vlieses. Ein derartiges Vlies besteht aus gerichteten oder ungerichteten, dreikomponentigen Fasern, wie sie beispielsweise in der Figur 4 beschrieben sind. Derartige triaxial elektrogesponnene Polymerfasern können ungerichtet oder in mehreren Schichten gerichtet auf einer Fläche abgeschieden werden, um ein Vlies zu erzeugen.

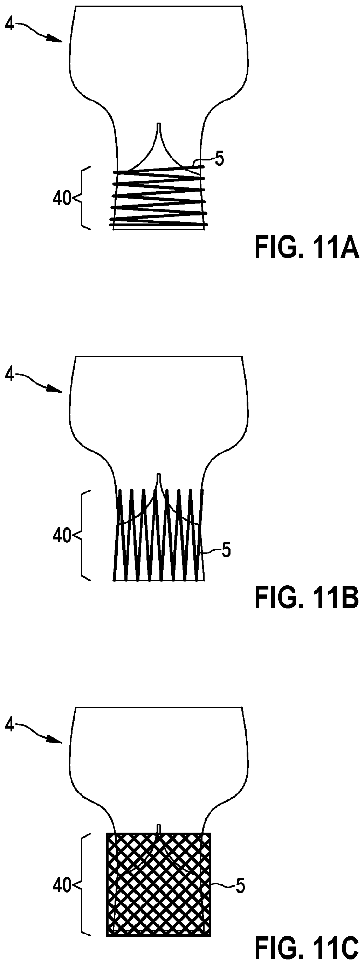

Die Figur 11A zeigt eine schematische Seitenansicht einer Herzklappenprothese 4, die an einem einflussseitigen Bereich 40 mit einem Dichtungsmaterial 5 versehen ist, das entsprechend der in den Figuren 1 bis 8 dargestellten Ausführungsbeispiele ausgestaltet sein kann. Das Dichtungsmaterial 5 weist also einen dreikomponentigen Aufbau auf. Es ist dabei als Faden oder als Tau ausgestaltet und auf einer Außenseite der Herzklappenprothese 4 um eine Stützstruktur der Herzklappenprothese 4 herumgewickelt. Damit ist dieses Dichtungsmaterial 5 besonders gut geeignet, die Herzklappenprothese 4 gegenüber einem kardialen Annulus eines Patienten, dem die Herzklappenprothese 4 implantiert wird, abzudichten.

Die Figur 11B zeigt ein weiteres Ausführungsbeispiel einer Herzklappenprothese 4, die an ihrem einflussseitigen Bereich 40 mit einem Dichtungsmaterial 5 versehen ist. Im Unterschied zur Figur 11A ist das Dichtungsmaterial 5 dabei nicht quer zu einer Längserstreckungsrichtung der Herzklappenprothese 4, sondern entlang einer Längserstreckungsrichtung der Herzklappenprothese 4 angeordnet. Auch in dieser Anordnung ist das Dichtungsmaterial 5 insbesondere dafür geeignet, die Herzklappenprothese 4 gegenüber einem kardialen Annulus eines Patienten, dem die Herzklappenprothese 4 implantiert wurde, abzudichten.

Die Figur 11C zeigt ein weiteres Ausführungsbeispiel einer Herzklappenprothese 4, bei der am einflussseitigen Bereich 40 ein flächiges Gebilde aus einem Dichtungsmaterial 5 angeordnet ist. Bei diesem flächigen Gebilde kann es sich insbesondere um einen Strick, ein Gewirk, eine Flechtung oder ein Vlies handeln, wie sie beispielsweise in den Figuren 9A bis 10 dargestellt sind. Durch die Anordnung des Dichtungsmaterials in Form eines derartigen flächigen Materials wird im Vergleich zu den in den Figuren 11A und 11B gezeigten Ausführungsbeispielen ein noch großflächigerer Bereich des einflussseitigen Bereichs 40 der Herzklappenprothese 4 von dem Dichtungsmaterial 5 bedeckt, sodass insgesamt eine noch stärkere Dichtung zwischen der Herzklappenprothese 4 und einer Gefäßwandung eines Patienten, dem die Herzklappenprothese 4 implantiert wurde, insbesondere gegenüber einem kardialen Annulus eines solchen Patienten, ergibt. Somit lässt sich das Risiko einer paravalvulären Leckage reduzieren oder gänzlich vermeiden. Durch das Dichtungsmaterial 5 kommt es zu einem Formschluss hin zur Gefäßwand des Patienten, beispielsweise zur aortalen Gefäßwand des Patienten.

Neben den in den Figuren 11A bis 11C gezeigten Ausführungsbeispielen, bei denen das Dichtungsmaterial 5 außen an der Herzklappenprothese 4 angeordnet ist, ist es grundsätzlich auch möglich, das Dichtungsmaterial zwischen Herzklappentaschen und einer Stentstruktur der Herzklappenprothese 4 anzubringen. Ferner ist es denkbar, dass das Dichtungsmaterial durch die einzelnen Zellen der Stentstruktur der Herzklappenprothese 4 hindurch geflochten ist. Auch kann das Dichtungsmaterial beispielsweise in Taschen, die auf einer Außenseite oder einer Innenseite einer Stentstruktur der Herzklappenprothese gebildet werden (beispielsweise durch eine innere Schürze oder eine äußere Schürze, die an der Stentstruktur angebracht sind), eingefüllt sein. Dann kann der Kontakt des Dichtungsmaterials mit einer wässrigen Flüssigkeit wie Blut zeitlich verzögert werden, da das Blut zunächst in die gebildeten Taschen eindringen muss, um in Kontakt mit dem Dichtungsmaterial zu treten.

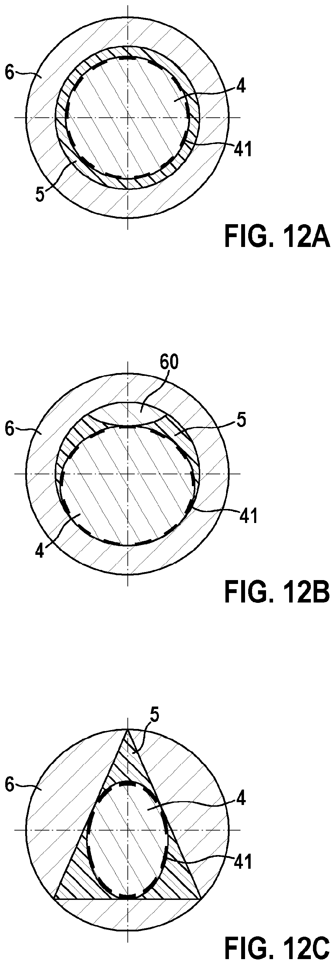

Die Figur 12A zeigt einen schematischen Querschnitt durch eine Aorta 6, in die eine Herzklappenprothese 4 implantiert wurde. Dabei weist die Herzklappenprothese 4 eine Stentstruktur 41 auf, an deren Außenseite die Herzklappenprothese 4 mit einem Dichtungsmaterial 5 versehen ist. Das Dichtungsmaterial ist - wie oben erläutert - ein Drei-Komposit-Material. Die Figur 12A zeigt dabei einen ideal runden Querschnitt der Aorta 6.

Demgegenüber zeigt die Figur 12B den Querschnitt durch eine Aorta 6, die eine Kalzifizierung 60 aufweist. Dadurch ergibt sich im Querschnitt eine ungleichmäßig implantierte Herzklappenprothese 4 und ein ungleichmäßig verteiltes Dichtungsmaterial 5. Gleichwohl ist das Dichtungsmaterial 5 sehr gut geeignet, die Herzklappenprothese 4 gegenüber der Kalzifizierung 60 abzudichten und somit für einen sicheren Sitz der Herzklappenprothese 4 innerhalb der Aorta 6 zu sorgen.

Die Figur 12C zeigt schließlich einen Querschnitt durch eine Aorta, die einen im Wesentlichen dreieckigen Querschnitt aufgrund zusammengewachsener Klappensegel aufweist. Dadurch ist die Herzklappenprothese 4 ebenfalls ungleichförmig implantiert, wobei das Dichtungsmaterial 5 die verbliebenen Bereiche zwischen Herzklappenprothese 4 und der Aorta 6 derart abdichtet, dass die Herzklappenprothese 4 sicher in der Aorta angeordnet ist und keine paravalvulären Leckagen auftreten.

Mithin eignet sich das Dichtungsmaterial 5 hervorragend zur Abdichtung von Herzklappenprothesen bei unterschiedlichen anatomischen Gegebenheiten bzw. Herausforderungen.

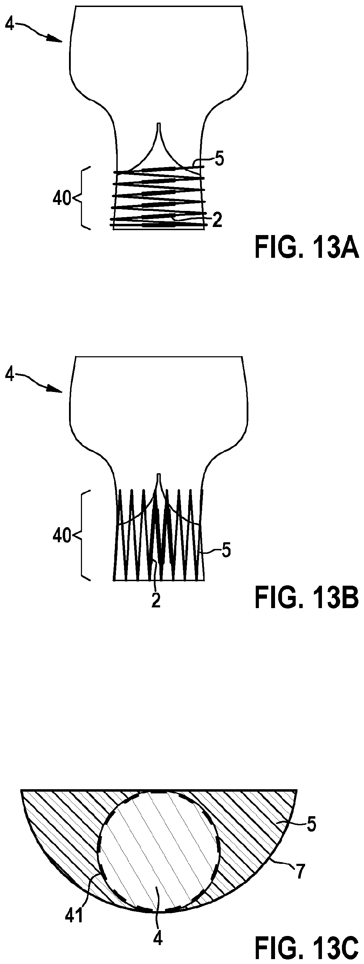

Die Figur 13A zeigt eine weitere schematische Darstellung einer Herzklappenprothese 4, die an ihrem einflussseitigen Bereich 40 ein Dichtungsmaterial 5 aufweist. Dabei ist das Dichtungsmaterial 5 in Form eines Tauwerks ausgestaltet, wobei die zweite Komponente 2 des Dichtungsmaterials 5 ortsaufgelöst an besonders relevanten Abschnitten der Herzklappenprothese 4 in größerem Maße vorhanden ist als in anderen Abschnitten der Herzklappenprothese 4. Dabei ist das Dichtungsmaterial 5 in dem Ausführungsbeispiel der Figur 13A - ähnlich wie beim Ausführungsbeispiel der Figur 11A - quer zu einer Längserstreckungsrichtung der Herzklappenprothese 4 angeordnet.

Die Figur 13B zeigt eine der Figur 13A vergleichbare Ausgestaltung einer Herzklappenprothese 4, wobei das Dichtungsmaterial 5 - ähnlich wie beim Ausführungsbeispiel der Figur 11B - entlang einer Längserstreckungsrichtung der Herzklappenprothese 4 angeordnet ist. Dabei weisen bestimmte Bereiche des Dichtungsmaterials 5 einen erhöhten Anteil der zweiten Komponente 2 auf und ermöglichen somit nach einem entsprechenden Kontakt mit einer wässrigen Lösung, wie einer Körperflüssigkeit, eine verstärkte Abdichtung zwischen der Herzklappenprothese 4 und einer die Herzklappenprothese 4 im implantierten Zustand umgebenden Gefäßwandung in diesen Bereichen.

Die Figur 13C zeigt eine schematische Querschnittsdarstellung durch einen mitralen Annulus, in den eine Herzklappenprothese 4 mit einer Stentstruktur 41 eingesetzt wurde. Dabei wird die Stentstruktur 41 von einem Dichtungsmaterial 5 umgeben, das die Herzklappenprothese 4 gegenüber dem mitralen Annulus 7 zuverlässig abdichtet. Zu diesem Zweck ist das Dichtungsmaterial 5, das Teil der Herzklappenprothese 4 ist, stark gegenüber seinem Ausgangszustand gequollen.

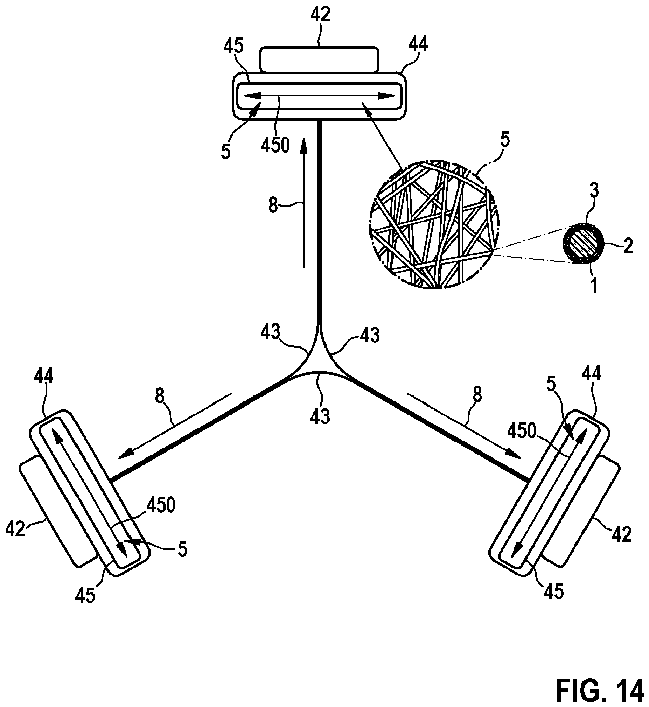

Die Figur 14 zeigt eine schematische Darstellung des Funktionsprinzips einer Straffung von Klappensegeln 43 einer Herzklappenprothese 4 durch Einbringung von Dichtungsmaterial 5 an einer Stützstruktur der Herzklappenprothese 4.

Wie oben erläutert, weisen Herzklappenprothesen 4 typischerweise eine Stentstruktur auf. An dieser Stentstruktur sind regelmäßig Kommissuren 42 zur Befestigung von Klappensegeln 43 vorgesehen. Im Ausführungsbeispiel der Figur 14 erfolgt die Befestigung der einzelnen Herzklappensegel 43 an den Kommissuren 42 über äußere Taschen 44, die in ihrem Inneren jeweils innere Taschen 45 aufweisen. Diese inneren Taschen 45 sind mit dem dreikomponentigen Dichtungsmaterial 5 entsprechend den obigen Erläuterungen gefüllt. Dabei ist das Dichtungsmaterial 5 insbesondere in Form eines quellbaren Flächenmaterials - beispielsweise in Form einer Vliesstruktur - in den inneren Taschen 45 angeordnet. Wie bereits in Zusammenhang mit den Figuren 1 bis 10 erläutert, besteht das Dichtungsmaterial 5 aus einer ersten Komponente 1, einer zweiten Komponente 2 und einer dritten Komponente 3.

Wenn das Dichtungsmaterial nun in Kontakt mit einer Körperflüssigkeit, insbesondere Blut, oder einer anderen wässrigen Flüssigkeit kommt, quellen zunächst die dritte Komponente 3 und anschließend die zweite Komponente 2 auf. Dabei sind die inneren Taschen 45 derart ausgebildet, dass sie eine Expansion des Dichtungsmaterials 5 in einer Längserstreckungsrichtung 450 erlauben, eine Expansion quer zu dieser Längserstreckungsrichtung 450 jedoch im Wesentlichen nicht gestatten.

Durch diese Ausgestaltung der inneren Taschen 45 kommt es bei einem Quellen des Dichtungsmaterials 5 folglich zu einer Zugkraft 8 auf die einzelnen Klappensegel 43, die in Richtung auf die Kommissuren 42 wirkt. Dadurch werden die Klappensegel 43 gestrafft.

Somit eignet sich das Dichtungsmaterial 5 nicht nur zur Abdichtung einer Herzklappenprothese gegenüber einer die Herzklappenprothese umgebenen Gefäßwand, sondern kann auch für andere Einsatzzwecke, wie beispielsweise die Straffung von Herzklappensegeln, eingesetzt werden. Auch in diesem Zusammenhang hat die zeitlich gestaffelte Quellung des Dichtungsmaterials 5 einen entscheidenden Vorteil. Denn so wirken die auf die Herzklappensegel 43 ausgeübten Kräfte zeitlich gestaffelt ein. Dadurch kann eine initiale Belastung der Herzklappensegel 43 reduziert werden und eine insgesamt bessere und nachhaltigere Straffung der Herzklappensegel 43 erreicht werden.

## Patentansprüche

1. Dichtungsmaterial für ein medizinisches Implantat,
**gekennzeichnet durch**
eine Kompositstruktur mit einer ersten Komponente (1), einer zweiten Komponente (2) und einer dritten Komponente (3), wobei
- die erste Komponente (1) mindestens ein biologisch inertes Polymer aufweist,
- die zweite Komponente (2) ein Hydrogel aufweist, das nach Kontakt mit einer wässrigen Lösung aufquillt und dadurch innerhalb einer ersten Zeitdauer eine erste Volumenvergrößerung erfährt, und
- die dritte Komponente (3) eine hygroskopische Matrix aufweist, die nach Kontakt mit einer wässrigen Lösung aufquillt und dadurch innerhalb einer zweiten Zeitdauer eine zweite Volumenvergrößerung erfährt,
wobei die zweite Zeitdauer kürzer als die erste Zeitdauer ist.

2. Dichtungsmaterial gemäß Anspruch 1, **dadurch gekennzeichnet, dass** das mindestens eine biologisch inerte Polymer eine Zugfestigkeit von 0,1 N/mm² bis 20 N/mm² und/oder eine Bruchdehnung von 30% bis 500% aufweist und/oder, dass das Hydrogel nach der ersten Volumenvergrößerung eine Zugfestigkeit von 0,02 N/mm² bis 1 N/mm² und/oder eine Bruchdehnung von 30% bis 130% aufweist und/oder, dass die hygroskopische Matrix nach der zweiten Volumenvergrößerung eine Zugfestigkeit von bis zu 2.5 N/mm² und/oder eine Bruchdehnung von 10% bis 40% aufweist.

3. Dichtungsmaterial gemäß Anspruch 1 oder 2, **dadurch gekennzeichnet, dass** die erste Zeitdauer 1 Stunde bis 10 Stunden beträgt und/oder die erste Volumenvergrößerung eine Vergrößerung eines initialen Volumens des Hydrogels um mindestens einen Faktor 2 bewirkt.

4. Dichtungsmaterial gemäß einem der vorhergehenden Ansprüche, **dadurch gekennzeichnet, dass** die zweite Zeitdauer 10 Sekunden bis 59 Minuten beträgt und/oder die zweite Volumenvergrößerung eine Vergrößerung eines initialen Volumens der hygroskopischen Matrix um mindestens einen Faktor 2 bewirkt.

5. Dichtungsmaterial gemäß einem der vorhergehenden Ansprüche, **dadurch gekennzeichnet, dass** das Hydrogel derart beschaffen ist, dass die erste Volumenvergrößerung durch einen externen Stimulus beschleunigt oder vergrößert wird.

6. Dichtungsmaterial gemäß einem der vorhergehenden Ansprüche, **dadurch gekennzeichnet, dass** das mindestens eine biologisch inerte Polymer ausgewählt ist aus der Gruppe bestehend aus Polyurethanen, Polyimiden, Polyethylenen, Polypropylenen, Polysulfonen, Polyestern, Polytetrafluoroethylen, Silikonen, Fluorosilikonen, Polyaryletherketonen, Polyvinylidenfluorid, Vinylpyrrolidon/ Vinylacetat-Copolymeren und Polyvinylfluorid.

7. Dichtungsmaterial gemäß einem der vorhergehenden Ansprüche, **dadurch gekennzeichnet, dass** das Hydrogel mindestens eine Substanz aufweist, die ausgewählt ist aus der Gruppe bestehend aus polymerisierbaren ionischen Flüssigkeiten, thermosensitiven Polymeren, Polyacrylamiden, Polyoxazolinen, Polyvinylethern und Polyethylenglykolen.

8. Dichtungsmaterial gemäß einem der vorhergehenden Ansprüche, **dadurch gekennzeichnet, dass** die hygroskopische Matrix mindestens eine Substanz aufweist, die ausgewählt ist aus der Gruppe bestehend aus Zellstoffmatrix, Cellulose-Derivaten und Chitosan.

9. Dichtungsmaterial gemäß einem der vorhergehenden Ansprüche, **dadurch gekennzeichnet, dass** die zweite Komponente (2) mindestens eine Substanz zur Förderung der Endothelialisierung aufweist.

10. Dichtungsmaterial gemäß einem der vorhergehenden Ansprüche, **dadurch gekennzeichnet, dass** die dritte Komponente (3) mindestens eine Substanz mit antikalzifizierender Wirkung aufweist.

11. Dichtungsmaterial gemäß einem der vorhergehenden Ansprüche, **dadurch gekennzeichnet, dass** das Dichtungsmaterial (5) einen Schichtaufbau aufweist, wobei die erste Komponente (1) einen Kernbereich des Dichtungsmaterials (5) bildet, die zweite Komponente (2) eine erste Schicht bildet, die den Kernbereich umgibt, und die dritte Komponente (3) eine zweite Schicht bildet, die die erste Schicht umgibt.

12. Dichtungsmaterial gemäß einem der Ansprüche 1 bis 11, **dadurch gekennzeichnet, dass** die zweite Komponente (2) und die dritte Komponente (3) als Kompositmaterial vorliegen.

13. Dichtungsmaterial gemäß einem der vorhergehenden Ansprüche, **dadurch gekennzeichnet, dass** das Dichtungsmaterial (5) in Form eines monofilen Fadens, eines multifilen Fadens, eines Tauwerks, eines Gestricks, eines Gewirks, einer Flechtung und/oder eines Vlieses vorliegt.

14. Medizinisches Implantat, **gekennzeichnet durch** ein Dichtungsmaterial (5) gemäß einem der vorhergehenden Ansprüche an zumindest einem Bereich (40) seiner Oberfläche, das im implantierten Zustand des medizinischen Implantats (4) dafür vorgesehen und eingerichtet ist, eine anatomische Struktur eines Patienten, insbesondere eine Gefäßwandung, insbesondere ein Gefäß, der/dem das medizinische Implantat (4) implantiert wurde, zu kontaktieren.

## Claims

1. Sealing material for a medical implant,
**characterized by**
a composite structure comprising a first component (1), a second component (2) and a third component (3), wherein
- the first component (1) comprises at least one biologically inert polymer,
- the second component (2) comprises a hydrogel which swells after contact with an aqueous solution and thereby undergoes a first volume increase within a first period of time, and
- the third component (3) has a hygroscopic matrix which swells after contact with an aqueous solution and thereby undergoes a second volume increase within a second period of time,
where the second duration is shorter than the first duration.

2. Sealing material according to claim 1, **characterized in that** the at least one biologically inert polymer has a tensile strength of 0.1 N/mm² to 20 N/mm² and/or an elongation at break of 30% to 500% and/or **in that** the hydrogel has a tensile strength of 0.02 N/mm² to 1 N/mm² and/or an elongation at break of 30% to 130% after the first volume increase and/or **in that** the hygroscopic matrix has a tensile strength of up to 2.5 N/mm² and/or an elongation at break of 10% to 40% after the second volume increase.

3. Sealing material according to claim 1 or 2, **characterized in that** the first period of time is 1 hour to 10 hours and/or the first volume increase causes an increase of an initial volume of the hydrogel by at least a factor of 2.

4. Sealing material according to one of the preceding claims, **characterized in that** the second time period is 10 seconds to 59 minutes and/or the second volume increase causes an increase of an initial volume of the hygroscopic matrix by at least a factor of 2.

5. Sealing material according to any one of the preceding claims, **characterized in that** the hydrogel is configured such that the first volume increase is accelerated or increased by an external stimulus.

6. Sealing material according to one of the preceding claims, **characterized in that** the at least one biologically inert polymer is selected from the group consisting of polyurethanes, polyimides, polyethylenes, polypropylenes, polysulfones, polyesters, polytetrafluoroethylenes, silicones, fluorosilicones, polyaryletherketones, polyvinylidene fluoride, vinylpyrrolidone/vinyl acetate copolymers and polyvinyl fluoride.

7. Sealing material according to any one of the preceding claims, **characterized in that** the hydrogel comprises at least one substance selected from the group consisting of polymerizable ionic liquids, thermosensitive polymers, polyacrylamides, polyoxazolines, polyvinyl ethers and polyethylene glycols.

8. Sealing material according to any one of the preceding claims, **characterized in that** the hygroscopic matrix comprises at least one substance selected from the group consisting of cellulose matrix, cellulose derivatives and chitosan.

9. Sealing material according to one of the preceding claims, **characterized in that** the second component (2) comprises at least one substance for promoting endothelialization.

10. Sealing material according to one of the preceding claims, **characterized in that** the third component (3) comprises at least one substance with an anticalcifying effect.

11. Sealing material according to any of the preceding claims, **characterized in that** the sealing material (5) has a layered structure, wherein the first component (1) forms a core region of the sealing material (5), the second component (2) forms a first layer surrounding the core region, and the third component (3) forms a second layer surrounding the first layer.

12. Sealing material according to any one of claims 1 to 11, **characterized in that** the second component (2) and the third component (3) are present as a composite material.

13. Sealing material according to one of the preceding claims, **characterized in that** the sealing material (5) is in the form of a monofilament yarn, a multifilament yarn, a cordage, a knitted fabric, a chrocheted fabric, a braid and/or a nonwoven fabric.

14. Medical implant, **characterized by** a sealing material (5) according to one of the preceding claims on at least one region (40) of its surface which, that in the implanted state of the medical implant (4), is intended and adapted to contact an anatomical structure of a patient, in particular a vessel wall, in particular a vessel on which the medical implant (4) has been implanted.

## Revendications

1. Matériau d'étanchéité pour un implant médical,
**caractérisé par**
une structure composite comprenant un premier composant (1), un deuxième composant (2) et un troisième composant (3), dans laquelle
- le premier composant (1) comprend au moins un polymère biologiquement inerte,
- le deuxième composant (2) présente un hydrogel qui gonfle après contact avec une solution aqueuse et subit ainsi une première augmentation de volume en l'espace d'une première durée, et
- le troisième composant (3) présente une matrice hygroscopique qui gonfle après contact avec une solution aqueuse et subit ainsi une deuxième augmentation de volume en l'espace d'une deuxième durée,
la deuxième durée étant plus courte que la première durée.

2. Matériau d'étanchéité selon la revendication 1, **caractérisé en ce que** ledit au moins un polymère biologiquement inerte présente une résistance à la traction de 0,1 N/mm² à 20 N/mm² et/ou un allongement à la rupture de 30% à 500% et/ou **en ce que** l'hydrogel présente, après la première augmentation de volume, une résistance à la traction de 0,02 N/mm² à 1 N/mm² et/ou un allongement à la rupture de 30% à 130% et/ou **en ce que** la matrice hygroscopique présente, après la deuxième augmentation de volume, une résistance à la traction allant jusqu'à 2,5 N/mm² et/ou un allongement à la rupture de 10% à 40%.

3. Matériau d'étanchéité selon la revendication 1 ou 2, **caractérisé en ce que** la première durée est de 1 heure à 10 heures et/ou la première augmentation de volume provoque une augmentation d'un volume initial de l'hydrogel d'au moins un facteur 2.

4. Matériau d'étanchéité selon l'une quelconque des revendications précédentes, **caractérisé en ce que** la seconde durée est comprise entre 10 secondes et 59 minutes et/ou **en ce que** la seconde augmentation de volume provoque une augmentation d'un volume initial de la matrice hygroscopique d'au moins un facteur 2.

5. Matériau d'étanchéité selon l'une quelconque des revendications précédentes, **caractérisé en ce que** l'hydrogel est tel que la première augmentation de volume est accélérée ou augmentée par un stimulus externe.

6. Matériau d'étanchéité selon l'une quelconque des revendications précédentes, **caractérisé en ce que** ledit au moins un polymère biologiquement inerte est choisi dans le groupe constitué par les polyuréthanes, les polyimides, les polyéthylènes, les polypropylènes, les polysulfones, les polyesters, le polytétrafluoroéthylène, les silicones, les fluorosilicones, les polyaryléthercétones, le fluorure de polyvinylidène, les copolymères de vinylpyrrolidone/acétate de vinyle et le fluorure de polyvinyle.

7. Matériau d'étanchéité selon l'une quelconque des revendications précédentes, **caractérisé en ce que** l'hydrogel comprend au moins une substance choisie dans le groupe constitué par les liquides ioniques polymérisables, les polymères thermosensibles, les polyacrylamides, les polyoxazolines, les polyvinyléthers et les polyéthylèneglycols.

8. Matériau d'étanchéité selon l'une quelconque des revendications précédentes, **caractérisé en ce que** la matrice hygroscopique comprend au moins une substance choisie dans le groupe constitué par la matrice de cellulose, les dérivés de cellulose et le chitosan.

9. Matériau d'étanchéité selon l'une des revendications précédentes, **caractérisé en ce que** le deuxième composant (2) comprend au moins une substance destinée à favoriser l'endothélialisation.

10. Matériau d'étanchéité selon l'une quelconque des revendications précédentes, **caractérisé en ce que** le troisième composant (3) comprend au moins une substance ayant un effet anticalcaire.

11. Matériau d'étanchéité selon l'une quelconque des revendications précédentes, **caractérisé en ce que** le matériau d'étanchéité (5) présente une structure en couches, le premier composant (1) formant une zone centrale du matériau d'étanchéité (5), le deuxième composant (2) formant une première couche entourant la zone centrale, et le troisième composant (3) formant une deuxième couche entourant la première couche.

12. Matériau d'étanchéité selon l'une quelconque des revendications 1 à 11, **caractérisé en ce que** le deuxième composant (2) et le troisième composant (3) sont présents sous forme de matériau composite.

13. Matériau d'étanchéité selon l'une quelconque des revendications précédentes, **caractérisé en ce que** le matériau d'étanchéité (5) se présente sous la forme d'un fil monofilament, d'un fil multifilament, d'un cordage, d'un tricot, d'une bonneterie, d'un tressage et/ou d'un non-tissé.

14. Implant médical, **caractérisé par** un matériau d'étanchéité (5) selon l'une des revendications précédentes, sur au moins une zone (40) de sa surface, qui, à l'état implanté de l'implant médical (4), est prévu et agencé pour entrer en contact avec une structure anatomique d'un patient, en particulier une paroi de vaisseau, notamment un vaisseau, sur laquelle/lequel l'implant médical (4) a été implanté.
